## Europäisches Patentamt
## European Patent Office
## Office européen des brevets

(19)

(11) Veröffentlichungsnummer: **0 158 872**
**B1**

(12)

# EUROPÄISCHE PATENTSCHRIFT

(45) Veröffentlichungstag der Patentschrift:
18.01.89

(21) Anmeldenummer: 85103461.1

(22) Anmeldetag: 23.03.85

(51) Int. Cl.⁴: **C 12 N 15/00**, C 12 N 1/00,
C 12 N 1/06 // C12R1:465

(54) Das Streptomyceten-Plasmid pSG5, Verfahren zu seiner Gewinnung und seine Verwendung.

(30) Priorität: 31.03.84 DE 3412092
31.03.84 DE 3412093

(43) Veröffentlichungstag der Anmeldung:
23.10.85 Patentblatt 85/43

(45) Bekanntmachung des Hinweises auf die Patenterteilung:
18.01.89 Patentblatt 89/3

(84) Benannte Vertragsstaaten:
AT BE CH DE FR GB IT LI LU NL SE

(56) Entgegenhaltungen:
EP-A- 0 066 701

(73) Patentinhaber: HOECHST AKTIENGESELLSCHAFT,
Postfach 80 03 20, D-6230 Frankfurt am Main 80 (DE)

(72) Erfinder: Wohlleben, Wolfgang, Dr.,
Schongauerstrasse 14, D-4800 Bielefeld 1 (DE)
Erfinder: Schulte, Agnes, Schlosshofstrasse 117,
D-4800 Bielefeld 1 (DE)
Erfinder: Pühler, Alfred, Prof. Dr., Am
Waldschlösschen 2, D-4800 Bielefeld 1 (DE)
Erfinder: Muth, Günter, Mergenthaler Weg 8,
D-4800 Bielefeld 1 (DE)

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft das neue Plasmid pSG5, das aus Streptomyces ghanaensis DSM 2932 isolierbar ist, sowie die Verwendung dieses Plasmids zur Herstellung von Plasmidvektoren, z.B. Pendelvektoren zwischen Streptomycetenstämmen und anderen Mikroorganismen.

Aus der europäischen Patentanmeldung mit der Veröffentlichungsnummer 66 701 ist das Streptomyceten-Plasmid pSG2 bekannt, das aus dem S. ghanaensis-Stamm ATCC 14672 isoliert wurde. Dieses Plasmid ist als endogenes Plasmid dazu geeignet, Plasmidvektoren zu konstruieren, die in S. ghanaensis eingesetzt werden sollen.

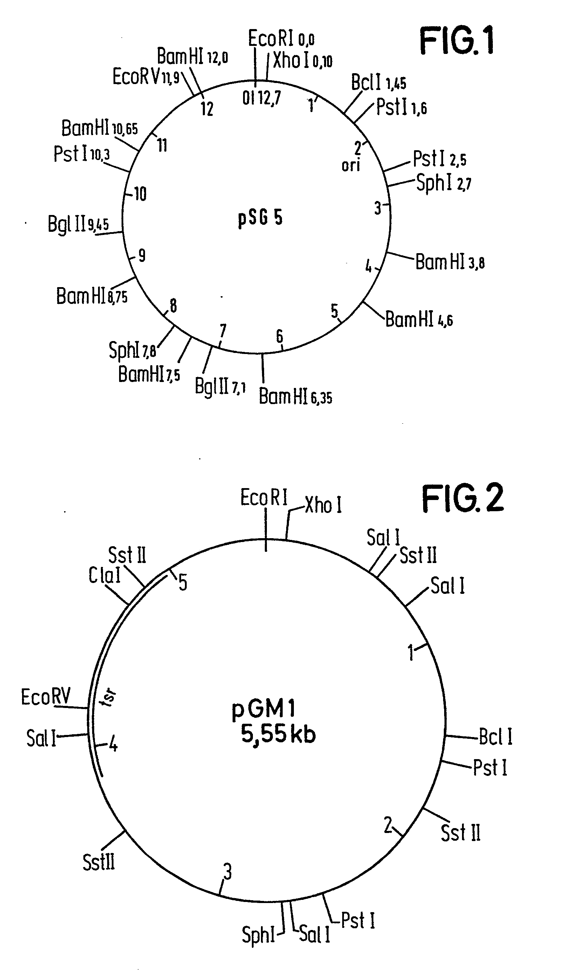

Es wurde nun gefunden, dass in dem S. ghanaensis-Stamm DSM 2932 ein Plasmid vorkommt, das als pSG5 bezeichnet wurde. Dieses Plasmid ist durch Restriktionsschnitte (Tabelle 1) und durch die Restriktionskarte (Figur 1) charakterisiert. Pro Zelle sind 10 bis 20 Plasmide enthalten. Das Plasmid weist eine Moleküllänge von etwa 12,7 Kilobasenpaaren (kb) auf und ist somit etwas kleiner als pSG2. Hybridisierungsdaten zeigen, dass zwischen pSG2 und pSG5 keine Verwandtschaft besteht.

Neben der etwas geringeren Grösse zeichnet sich pSG5 im Vergleich zu pSG2 durch mindestens vier singuläre Schnittstellen für die Restiktionsenzyme EcoRI, EcoRV, XhoI und BclI aus. Es bestehen somit mehrere Möglichkeiten, dieses Plasmid für gentechnologische Zwecke einzusetzen, z.B. zur Konstruktion von Plasmidvektoren.

Es wurde weiterhin gefunden, dass pSG5 einen erweiterten Wirtsbereich aufweist. Aus pSG5 hergestellte Plasmidvektoren können beispielsweise nach S. lividans und S. geysirensis transformiert werden.

Ein weiterer Vorteil ist, dass pSG5 mit anderen Streptomyceten-Replikons kompatibel ist, beispielsweise mit den Plasmiden pSG2 aus S. ghanaensis, pSVH1 (europäische Patentanmeldung mit der Veröffentlichungsnummer 70 522) aus S. venezuelae DSM 40755, SLP1.2 (US-Patentschrift 4 360 597, C. J. Thompson, J. M. Ward and D. A. Hopwood, Nature 286 (1980) 525; C. J. Thompson, T. Kieser, J. M. Ward and D. A. Hopwood, Gene 20 (1982) 51) aus S. lividans und pIJ101 (T. Kieser, D. A. Hopwood, H. M. Wright und C. J. Thompson, Mol. Gen. Genet. 185 (1982) 223) aus S. lividans. pSG5 kann somit zusätzlich in Stämme eingeführt werden, die bereits eines dieser Plasmide tragen.

Dies ist von Bedeutung, wenn man einzelne Gene in unterschiedliche Plasmide kloniert und in ein und dieselbe Zelle einführen will. Das Vorhandensein kompatibler Vektorplasmide unterschiedlicher Kopienzahl erlaubt darüber hinaus die Kombination einzelner Gene in unterschiedlicher Anzahl in einem Streptomyceten-Stamm. Sowohl für das Studium von Biosynthesewegen als auch für Ausbeuteoptimierung ist die Existenz von Plasmidvektoren unterschiedlicher Kompatibilität von entscheidender Bedeutung.

Durch Transposon-Mutagenese und Klonierungen wurde gefunden, dass die Replikationsregion auf dem grössten BamHI-Fragment (4,45 kb) liegt, und zwar in dem Bereich von 1 bis 3 kb entsprechend Figur 1. Zur Konstruktion von Vektoren, die in Streptomyceten replizieren, genügt somit diese «essentielle Region».

Zur Konstruktion eines «Minimalreplicons» bietet sich z.B. das genannte 4,45 kb lange BamHI-Fragment an. Zirkularisiert man dieses Fragment, so erhält man ein Plasmid, das durch singuläre Schnittstellen für BclI, SphI, BamHI, EcoRI und XhoI ausgezeichnet ist. Für die vier letztgenannten Schnittstellen ist gezeigt, dass sie für Klonierungen zur Verfügung stehen. Darüber hinaus liegen auf diesem Fragment noch Schnittstellen für PvuII (bei 12,6, 3,2 und 3,6 kb gemäss Figur 1), die ausserhalb der «essentiellen Region» liegen und somit für Klonierungen oder weitere Verkürzung(en) zur Verfügung stehen. Es wurden weiterhin Schnittstellen für SstII (bei 0,6, 1,8 und 3,65 kb gemäss Figur 1) gefunden, von denen die mittlere vermutlich in der «essentiellen Region» liegt. Ausserdem sind mindestens fünf SalI-Schnittstellen vorhanden.

Wird das 4,45 kb BamHI-Fragment mit dem 1,1 kb BclI-Fragment aus dem Plasmid pIJ6 (Thompson et al., Nature, a.a.O.) mit dem Thiostrepton-Resistenzgen (tsr) ligiert, so erhält man das Hybridplasmid pGM1, das in Streptomyceten repliziert und aufgrund seiner Thiostrepton-Resistenz leicht selektioniert werden kann. Insbesondere die singulären Schnittstellen für EcoRI und XhoI stehen für den Einbau von Fremdgenen zur Verfügung. Die Figur 2 zeigt die Restriktionskarte von pGM1, (in der im Replicon nur drei SalI-Schnittstellen kartiert sind).

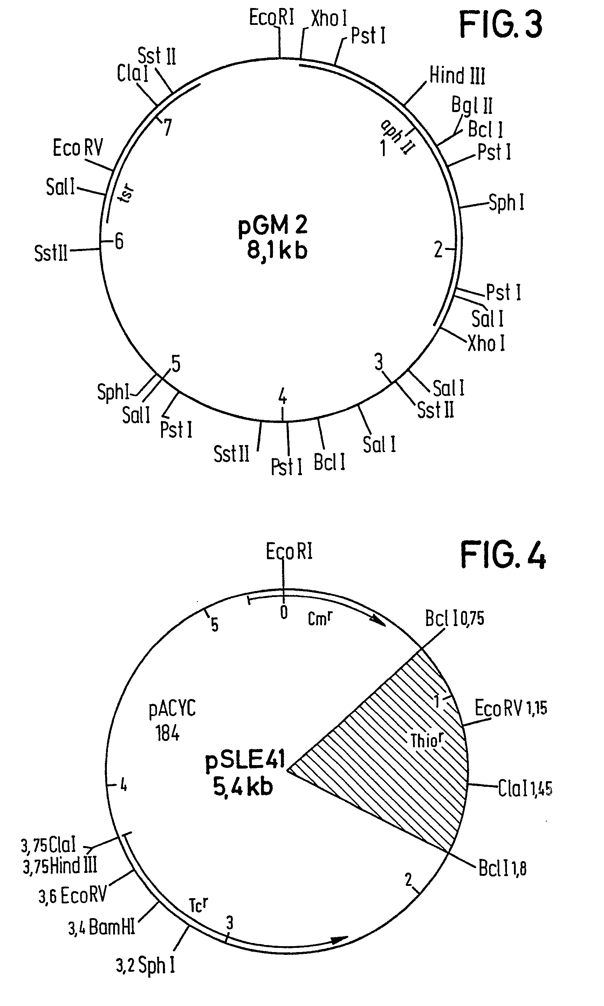

Wird beispielsweise das Plasmid pGM1 mit XhoI geöffnet und mit dem 2,55 kb XhoI-Fragment aus Tn5 (Jorgensen et al., Mol. gen. Genet. 177 (1977) 65; F.J. de Bruijn und J.R. Lupski, Gene 27 (1984) 131) ligiert, welches das in Streptomyceten und in E. coli wirksame Neomycin-Resistenzgen aphII trägt, so erhält man das Hybridplasmid pGM2 (Figur 3, in der im Replicon nur drei SalI-Schnittstellen kartiert sind). Dieses Plasmid weist also zwei in Streptomyceten effektive Marker auf und ist mit seiner Molekülgrösse von 8,1 kb und singulären Schnittstellen für EcoRI, HindIII, BglII, ClaI und EcoRV ein guter Klonierungsvektor. Die BglII-Schnittstelle liegt im aphII-Gen, ClaI und EcoRV schneiden innerhalb des tsr-Gens. Klonierungen mit diesen Enzymen können also leicht durch Insertionsinaktivierung verifiziert werden.

Das Plasmid pSG5 und seine Verkürzungen, die die «essentielle Region» enthalten, sind auch zur Konstruktion von sogenannten Pendelvektoren geeignet. Fusioniert man beispielsweise das Plasmid pSG5 mit den E. coli-Plasmiden pACYC184 oder pBR325, so kann das Hybridplasmid sowohl in E. coli als auch in Streptomyces-Arten, insbesondere in S. geysirensis und S. lividans, vermehrt werden.

Wird das Plasmid pACYC184 (Chang et al., J. Bacteriol. 134 (1978) 1141) mit BclI geöffnet und das linearisierte Plasmid mit dem 1,1 kb BclI-

Fragment aus dem Plasmid pIJ6 ligiert, so erhält man das Hybridplasmid pSLE41.

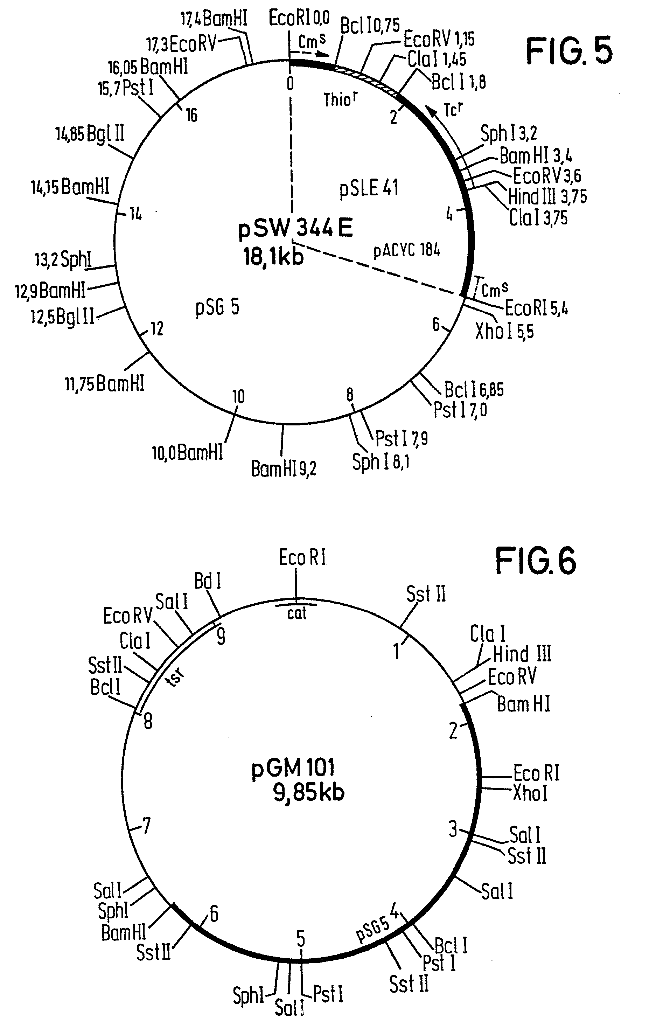

Werden beispielsweise die Plasmide pSG5 und pSLE41 über ihre EcoRI-Schnittstellen fusioniert, so erhält man das Hybridplasmid pSW344E (Figur 5), das sowohl in E. coli auf Grund des pACYC184-Replikons als auch in Streptomyceten auf Grund des pSG5-Replikons stabil repliziert wird. Die Selektion in E. coli erfolgt über die Tetracyclin-Resistenz des pACYC184-Anteils, während zur Selektion in Streptomyceten die Thiostreptonresistenz dienen kann. Das Plasmid pSW344E stellt somit einen idealen Pendelvektor zwischen E. coli und Streptomycetenstämmen dar. Zur Klonierung von Fremd-DNA stehen u.a. die Restriktionsstellen XhoI, HindIII und BglII zur Verfügung. Der Vorteil des Pendelvektors besteht darin, dass ein kloniertes Streptomycetengen in E. coli gentechnologisch verändert und seine Funktion nach Rücktransfer in der Streptomycetenzelle ausgetestet werden kann.

Wird pSLE41 mit BamHI geöffnet und mit dem 4,45 kb-BamHI-Fragment von pSG5 ligiert, so resultiert das Hybridplasmid pGM101 (Figur 6, in der im Replicon nur drei SalI-Schnittstellen kartiert sind). Dieses Plasmid verfügt über singuläre Schnittstellen für HindIII und XhoI in nichtessentiellen Regionen. Zur Selektion stehen die Marker Chloramphenicol-Resistenz (in E. coli) und Thiostrepton-Resistenz (in Streptomyces) zur Verfügung. Die Molekülgrösse beträgt 9,9 kb.

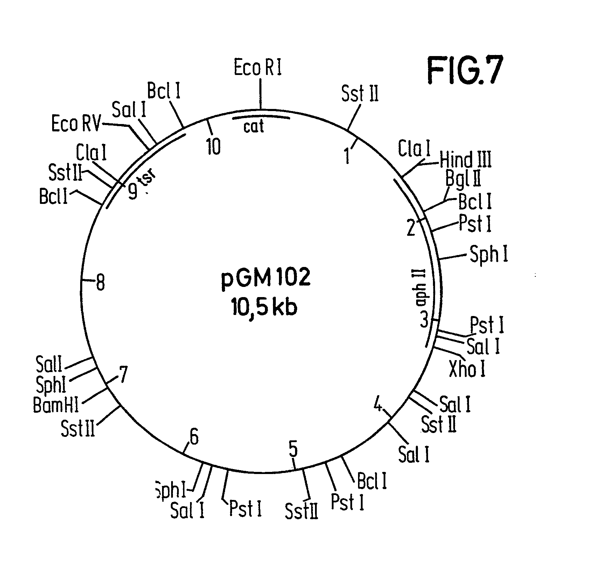

Eliminiert man aus pGM101 das 1 kb HindIII-XhoI-Fragment und inseriert statt dessen das 1,6 kb HindIII-XhoI-Fragment von Tn5, das das aphII-Resistenzgen für Neomycin trägt, so entsteht das Hybridplasmid pGM102 (Figur 7, in der im Replicon nur drei SalI-Schnittstellen kartiert sind). Dieses repliziert in E. coli und S. lividans. Zur Selektion in E. coli stehen die Chloramphenicol- und die Neomycinresistenz, in S. lividans die Thiostrepton- und die Neomycinresistenz zur Verfügung. Zur Klonierung können in E. coli die singulären Schnittstellen für EcoRI (Inaktivierung der Chloramphenicol-Resistenz) und BglII (Inaktivierung der Neomycin-Resistenz), in Streptomyceten die für EcoRV (Inaktivierung der Thiostrepton-Resistenz) und ebenfalls BglII dienen, ausserdem die singulären Schnittstellen für HindIII und XhoI.

Pendelvektoren dieser Art sind Gegenstand der am gleichen Tage eingereichten deutschen Patentanmeldung P 34 12 093.9 mit der Bezeichnung «Hybyridplasmide mit einem Streptomyceten- und einem Escherichia coli-Replicon». Sie eignen sich zur Herstellung von Antibiotika, Enzymen oder biologisch aktiven Polypeptiden, wie es für Streptomyceten-Plasmide in den US-Patentschriften 4 273 875, 4 332 900, 4 338 400, 4 340 674, 4 343 906, 4 362 816, 4 362 817, 4 393 137, 4 401 761 und 4 416 994 beschrieben ist.

Die Isolierung des Plasmids pSG5 aus dem Stamm S. ghanaensis DSM 2932 kann in an sich bekannter Weise erfolgen, wie es beispielsweise in den europäischen Patentanmeldungen mit den Veröffentlichungsnummern 66 701 und 70 522 beschrieben ist. Die Kultivierung des Stammes kann gemäss der erstgenannten europäischen Patentanmeldung bzw. US-Patentschrift 3 674 866, Beispiel 2, erfolgen.

In den folgenden Beispielen wird die Erfindung näher erläutert. Prozentangaben beziehen sich hierbei auf das Gewicht, sofern nichts anderes angegeben ist.

Beispiel 1
Stammkultivierung

Zur Kultivierung für eine anschliessende Lyse sind folgende Medien geeignet:

| Lysemedium A | | Lysemedium B | | Lysemedium C | |
|---|---|---|---|---|---|
| Glucose | 10 g | Yeast Extr. | 3 g | CASO-Bouillon | |
| Pepton | 4 g | Pepton | 5 g | (Merck # 5459) | 30 g |
| Yeast Extr. | 4 g | Malzextr. | 3 g | Glycin | 10 g |
| $KH_2PO_4$ | 2 g | Glucose | 10 g | $H_2O$ | 1 g |
| $K_2HPO_4$ | 4 g | Sucrose | 340 g | | |
| $MgSO_4$ | 0,5 g | Glycin | 5 g | | |
| Glycin | 10 g | $MgCl_2 \cdot 6H_2O$ | 1 g | | |
| $H_2O$ | 1 l | $H_2O$ | 1 l | | |

In einem 500 ml Erlenmeyerkolben werden ca. 100 ml Nährmedium mit einer homogenisierten Einzelkolonie beimpft und 2–3 Tage bei 30°C im Rundschüttler (120 rpm) inkubiert.

Beispiel 2
Plasmidisolierung

Ca. 50 ml einer 3 Tage alten, homogenisierten Flüssigkultur werden in JA-20 Bechern einer Beckman-Kühlzentrifuge J21C geerntet (10 min, 10 000 rpm, 25°C) und einmal in TESu (10 mM Tris-HCl, 1 mM EDTA (pH 8), 10% Sucrose) gewaschen. 2 g Zellen werden in 5 ml Lysozymlösung resuspendiert (0,3 M Sucrose, 25 mM Tris-HCl (pH 8), 25 mM EDTA (pH 8), 4 mg/ml Lysozym) und bei 37°C im Rundschüttler (100 rpm) inkubiert. Nach 30–45 min sind die Zellen protoplastiert. Durch Zugabe von 2,5 ml Lysemix (0,3 M NaOH, 2% Natrium-Dodecylsulfat) und sofortiges kräftiges Mischen erfolgt die Auflösung der Zellmembran und eine Denaturierung der DNA. Eine 10minütige Hitzebehandlung (70°C) komplettiert die Lyse und Denaturierung. Bei Raumtemperatur werden 800 µl saure Phenol/Chloroform-Lösung zugegeben (Herstellung der sauren Phenol/Chloroform-Lösung: 500 ml Chloroform, 200 ml $H_2O$,

500 g Phenol, 0,5 g Hydroxychinolin mischen und untere Phase benutzen), um die Proteine zu denaturieren und die DNA zu renaturieren. Das Gemisch wird ca. 20 sec in einem Schüttler (®VORTEX) durchmischt und dann in der Kühlzentrifuge pelletiert (15 min, 12 000 rpm, 4 °C).

7 ml des plasmidhaltigen Überstands werden dann in der Ultrazentrifuge weitergereinigt: 7 g CsCl, 7 ml Lysat und 0,2 ml Ethidiumbromidlösung (30 mg/ml) werden gemischt und in einer Ultrazentrifuge (®KONTRON TGA50) 48 h bei 34 000 rpm (20 °C) zentrifugiert. Die Plasmidbande wird nach UV-Bestrahlung über Fluoreszenz sichtbar gemacht und mit der Spritze entnommen. Die Lösung wird entfärbt und dialysiert und steht dann für weitere Untersuchungen zur Verfügung. Sie enthält ca. 1 μg Plasmid-DNA/20 μl Lösung.

Beispiel 3
Plasmidcharakterisierung

Die pSG5-DNA wird nach Darstellung im Elektronenmikroskop vermessen. Die Präparation erfolgt mit den bekannten Methoden (J. Ferguson, R.W. Davis in «Advanced Techniques in Biological Electron Microscopy II», Springer-Verlag, Berlin (1978) 123). Die Längenmessung ergibt ein Plasmid von 4,2 μm.

Um die Zahl der Restriktionsstellen für verschiedene Enzyme zu bestimmen, wird pSG5-DNA mit verschiedenen Restriktionsendonukleasen unter entsprechenden Inkubationsbedingungen 1 h lang behandelt (s. Tabelle 2).

Der Reaktionsansatz erfolgt in einem Gesamtvolumen von 20 μl, und zwar in folgender Zusammensetzung:

2 μl 10-fach konzentrierter Inkubationspuffer
x μl DNA-Lösung (enthält ca. 0,5 μg DNA)
1 μl Restriktionsenzym (enthält 1 unit)
17-x μl H$_2$O

Die Reaktion wird durch Zugabe von 5 μl Bromphenolblaulösung gestoppt. Die Fragmente werden durch Agarosegelelektrophorese aufgetrennt (1% Agarose in Trisacetatpuffer (0,04 M Trisacetat, 0,002 M EDTA)). Bei einer Laufzeit von 4 h beträgt die Spannung 4 Volt/cm Gellänge. Durch Vergleich mit einem Längenstandard (λ-DNA; EcoRI, HindIII gespalten, Längen bekannt (Phillipsen und Davis; Focus 1 (1979) 5)) kann die Fragmentlänge errechnet werden.

Aus den Restriktionsanalysen erhält man neben der Zahl der Schnittstellen die Fragmentlängen sowie die Gesamtlänge des Plasmids. Durch geeignete Mehrfachverdauungen lässt sich schliesslich die Restriktionskarte erstellen.

Beispiel 4
Konstruktion von Hybridplasmiden
a) Konstruktion von pSLE41

pACYC184-DNA kann aus plasmidtragenden Zellen nach den bekannten Verfahren (Maniatis et al., Molecular Cloning, Cold Spring Harbor 1982) gewonnen werden. Wildtyp-E. coli-Zellen besitzen allerdings eine Dam-Methylase, die die DNA so modifizieren, dass die erforderliche Restriktion mit BclI nicht möglich ist. Zur DNA-Isolierung wird in diesem Falle also eine Dam⁻-Mutante von E. coli verwendet.

Das Plasmid pIJ6 kann aus Streptomyces lividans TC14 nach den bekannten Streptomyceten-Techniken isoliert werden (siehe oben).

Zur Klonierung wird das Plasmid pACYC184 mit dem Restriktionsenzym BclI linearisiert.

1 μg DNA wird im Spaltungspuffer in Gegenwart von 1 unit BclI (Hersteller: BRL, Neu-Isenburg) 1 h bei 50 °C inkubiert. Die Reaktion wird durch Phenolisierung gestoppt und die DNA durch Ethanolfällung gereinigt. Eine anschliessende Behandlung mit alkalischer Phosphatase (aus Kälberdarm, Hersteller: Boehringer Mannheim) entfernt die 5′-Phosphatenden der DNA und verhindert die Religierung von pACYC184. Zur Gewinnung des DNA-Fragments, das die Thiostreptonresistenz trägt, wird pIJ6-DNA mit BclI geschnitten (Verfahren s.o.).

Die beiden DNA-Proben (im Spaltungspuffer) werden gemischt (0,1 μg pACYC184, 1 μg pIJ6) und auf 70 °C erhitzt, um H-Brücken zu öffnen. Durch Zugabe von Mercaptoethanol (Endkonz. 10 mM) und ATP (0,1 mM) werden die Reaktionsbedingungen eingestellt. Die DNA wird in Gegenwart von 1 unit T4-DNA-Ligase (Boehringer Mannheim) bei 4 °C für 12 h inkubiert.

Mit dem DNA-Gemisch wird E. coli transformiert (Maniatis et al.) und die Zellen auf Tetracyclin- und Chloramphenicolresistenz selektioniert.

Einzelkolonien werden einer Schnellyse zur Grössenbestimmung unterworfen. Im Agarosegel wird ein Plasmid der Grösse 5,4 kb (pACYC 184 4,3 kb + Thio$^r$ 1,1 kb) gesucht. Aus Zellen, die ein Plasmid der geforderten Länge tragen, wird DNA isoliert (s. oben) und durch eine Restriktionsanalyse geklärt, ob das richtige DNA-Fragment inseriert ist. Die Restriktionsschnitte des Fragments sind bekannt (Kieser et al.), so dass die erfolgreiche Klonierung verifiziert werden kann.

b) Herstellung des Plasmids pSW344E

Das Plasmid pSLE41 kann aus E. coli nach den bekannten Verfahren (Maniatis et al.) gewonnen werden. Das Plasmid pSG5 wird aus S. ghanaensis 2932 isoliert wie oben beschrieben.

Zur Klonierung werden beide Plasmide parallel mit EcoRI linearisiert. 1 μg DNA wird im Spaltungspuffer in Gegenwart von 1 unit EcoRI (Hersteller: Boehringer Mannheim) 1 h bei 37 °C inkubiert. Die Reaktion wird durch Phenolisierung gestoppt und die DNA durch Ethanolfällung gereinigt. pSLE41-DNA wird zweckmässigerweise noch mit alkalischer Phosphatase behandelt, um die Religierung zu unterbinden. Die DNA-Proben (im Spaltungspuffer) werden dann gemischt (0,2 μg pSLE41, 1 μg pSG5), auf 70 °C erhitzt und durch Zugabe von Mercaptoethanol (Endkonz. 10 mM) und ATP (0,1 mM) auf die Ligase-Reaktionsbedingungen eingestellt. In Gegenwart von 1 unit T4-DNA-Ligase (Boehringer Mannheim) wird das Gemisch 12 h bei 4 °C inkubiert. Anschliessend wird das Gemisch nach E. coli transformiert (Maniatis et al.) und auf Kolonien mit Tetracyclinresistenz und Chloramphenicolsensitivität selektioniert.

Kolonien mit entsprechendem Resistenzmuster werden einer Schnellyse zur Grössenbestimmung unterworfen. Aus Zellen, die ein Plasmid der geforderten Länge (18,1 kb) enthalten, wird DNA isoliert und durch Restriktionsanalyse geklärt, ob die DNA das entsprechende Restriktionsmuster liefert, das bei einer Ligierung von pSLE41 und pSG5 gefordert werden muss.

In ähnlicher Weise können die Hybridplasmide pGM1, pGM2, pGM101 und pGM102 hergestellt werden

Beispiel 5
Konstruktion eines Minimalreplikons aus pSG5

Das Plasmid pSG5 wird aus S. ghanaensis DSM 2932 wie oben beschrieben isoliert. Das Plasmid pSLE41 kann aus E. coli nach den bekannten Verfahren (Maniatis et al.) gewonnen werden.

In pSLE41 werden Fragmente von pSG5 kloniert, und zwar die Sphl-, BamHI- und BglII-Fragmente in die Sphl-, BamHI- und BamHI-Schnittstelle des pSLE41. Zur Klonierung werden beide Plasmide parallel mit den obengenannten Enzymen linearisiert (Hersteller und Inkubationsbedingung s. Tabelle 2). Die Reaktion wird durch Phenolisierung gestoppt und die DNA durch Ethanolfällung gereinigt (Maniatis et al.).

pSLE41-DNA wird zweckmässigerweise noch mit alkalischer Phosphatase behandelt, um die Religierung zu unterbinden (Maniatis et al.).

Die DNA-Proben (im Spaltungspuffer) werden dann gemischt (0,2 µg pSLE41, 1 µg pSG5), auf 70 °C erhitzt und durch Zugabe von Mercaptoethanol (Endkonz. 10 mM) und ATP (0,1 mM) auf die Ligase-Reaktionsbedingungen eingestellt. In Gegenwart von 1 unit T4-DNA-Ligase (Boehringer Mannheim) wird das Gemisch 12 h bei 4 °C inkubiert. Anschliessend wird das Gemisch nach E. coli transformiert (Maniatis et al.) und auf Kolonien mit Chloramphenicolresistenz und Tetracyclinsensitivität selektioniert. Kolonien mit entsprechendem Resistenzmuster werden einer Schnelllyse zur Grössenbestimmung (T. Eckhardt, Plasmid 1 (1978) 584) unterworfen. Aus Zellen, die ein Plasmid der geforderten Länge enthalten, wird DNA isoliert und durch Restriktionsanalyse geklärt, ob die erwarteten Fusionsplasmide entstanden sind.

Diese Plasmide werden mit den üblichen Verfahren nach S. lividans TK 23 transformiert (K.F. Chater, D.A. Hopwood, T. Kieser und C.J. Thompson: Gene Cloning in Streptomyces, Current Topics in Microbiol. and Immunol. 96, 69–95 (1982)) und auf Thiostreptonresistenz selektioniert. Aus thiostreptonresistenten S. lividans-Kolonien isoliert man Plasmid-DNA mit Standardprozeduren (T. Kieser, Factors Affecting the Isolation of ccc DNA from Streptomyces lividans and E. coli, Plasmid 12, 19 (1984)) und prüft ihre Zusammensetzung.

Alle Plasmide, die aus thiostreptonresistenten Kolonien erhalten werden, tragen die Gene des pSG5-Plasmids, die zur Replikation benötigt werden. So lässt sich aus dem Gesamtergebnis ein zweckmässiges «Minimalreplikon» identifizieren (hier das 4,45 kb BamHI-Fragment), das durch andere Klonierungsexperimente und durch vergleichende überlappende Klonierung weiter minimiert werden kann.

Tabelle 1
Anzahl der Schnittstellen im pSG5-Plasmid für ausgewählte Restriktionsenzyme

| Bgll | 0 |
|---|---|
| Hpal | 0 |
| HindIII | 0 |
| Clal | 0 |
| EcoRV | 1 |
| EcoRI | 1 |
| Xhol | 1 |
| Bcll | 1 |
| Sphl | 2 |
| BglII | 2 |
| Pstl | 3 |
| Sall | > 5 |
| SstII | > 5 |
| Smal | > 6 |
| BamHI | ≥ 7 |

Tabelle 2
Restriktionsbedingungen

| Enzym | Inkubations-temperatur in °C | Hersteller-firma | Inkubations-puffer |
|---|---|---|---|
| BamHI | 37 | Boe | |
| Bgll | 37 | Boe | |
| BglII | 37 | BRL | |
| Bcll | 60 | Boe | |
| EcoRI | 37 | Boe | |
| EcoRV | 37 | Boe | jeweils entsprechend der Firmen-empfehlung |
| Clal | 37 | BRL | |
| HindIII | 37 | Boe | |
| Hpal | 37 | Boe | |
| Kpnl | 37 | BRL | |
| Pstl | 37 | Boe | |
| Sall | 37 | BRL | |

Tabelle 2 (Fortsetzung)
Restriktionsbedingungen

| Enzym | Inkubations-temperatur in °C | Hersteller-firma | Inkubations-puffer |
|---|---|---|---|
| SmaI | 37 | Boe | |
| SphI | 37 | Boe | |
| SstI | 37 | BRL | |
| XhoI | 37 | BRL | |

Boe = Boehringer Mannheim, Mannheim
BRL = BRL, Neu-Isenburg

**Patentansprüche für die Vertragsstaaten: BE, CH, DE, FR, GB, IT, LI, LU, NL, SE**

1. Plasmid pSG5 mit einer Moleküllänge von etwa 12,7 kb, das von den Restriktionsenzymen BglI, HpaI, HindIII und ClaI nicht, von EcoRV, EcoRI, XhoI und BclI einmal und von SphI und BglII zweimal geschnitten wird, erhältlich aus einer Kultur von Streptomyces ghanaensis DSM 2932.

2. Verfahren zur Isolierung des Plasmids pSG5 gemäss Anspruch 1, dadurch gekennzeichnet, dass man eine Kultur von Streptomyces ghanaensis DSM 2932 lysiert und das Plasmid aus dem Lysat gewinnt.

3. Verwendung des Plasmids pSG5 gemäss Anspruch 1 oder seiner die intakte Replikationsregion enthaltenden Fragmente zur Konstruktion von Plasmidvektoren.

4. Verwendung des Plasmids pSG5 gemäss Anspruch 1 oder seiner die intakte Replikationsregion enthaltenden Fragmente zur Konstruktion von Plasmidvektoren für Streptomyceten.

5. Verwendung des Plasmids pSG5 gemäss Anspruch 1 oder seiner die intakte Replikationsregion enthaltenden Fragmente zur Konstruktion von Pendelvektoren zwischen Streptomycetenstämmen und anderen Mikroorganismen.

6. Hybridplasmide, die das Plasmid pSG5 gemäss Anspruch 1 oder seine intakte Replikationsregion enthalten.

7. Streptomyces ghanaensis DSM 2932.

**Patentansprüche für den Vertragsstaat AT:**

1. Verfahren zur Isolierung des Plasmids pSG5 mit einer Moleküllänge von etwa 12,7 kb, das von den Restriktionsenzymen BglI, HpaI, HindIII und ClaI nicht, von EcoRV, EcoRI, XhoI und BclI einmal und von SphI und BglII zweimal geschnitten wird, dadurch gekennzeichnet, dass man eine Kultur von Streptomyces ghanaensis DSM 2932 lysiert und das Plasmid aus dem Lysat gewinnt.

2. Verwendung des Plasmids pSG5 gemäss Anspruch 1 oder eines das intakte Replicon enthaltenden Fragments dieses Plasmids zur Konstruktion von Plasmidvektoren.

3. Verwendung des Plasmids pSG5 gemäss Anspruch 1 oder eines das intakte Replicon enthaltenden Fragments dieses Plasmids zur Konstruktion von Plasmidvektoren für Streptomyceten.

4. Verwendung des Plasmids pSG5 gemäss Anspruch 1 oder eines das intakte Replicon enthaltenden Fragments dieses Plasmids zur Konstruktion von Pendelvektoren zwischen Streptomycetenstämmen und anderen Mikroorganismen.

**Claims for the contracting states: BE, CH, DE, FR, GB, IT, LI, LU, NL, SE**

1. The plasmid pSG5 having a molecular length of about 12.7 kb, which is not cut by the restriction enzymes BglI, HpaI, HindIII and ClaI, is cut once by EcoRV, EcoRI, XhoI and BclI, and is cut twice by SphI and BglII, and is obtainable from a culture of Streptomyces ghanaensis DSM 2932.

2. A process for the isolation of the plasmid pSG5 according to claim 1, characterized by lysing a culture of Streptomyces ghanaensis DSM 2932 and obtaining the plasmid from the lysate.

3. The use of the plasmid pSG5 according to claim 1, or of its fragments containing the intact replication region, for the construction of plasmid vectors.

4. The use of the plasmid pSG5 according to claim 1, or of its fragments containing the intact replication region, for the construction of plasmid vectors for Streptomycetes.

5. The use of the plasmid pSG5 according to claim 1, or of its fragments containing the intact replication region, for the construction of shuttle vectors between Streptomycetes strains and other microorganisms.

6. Hybrid plasmids which contain the plasmid pSG5 according to claim 1 or its intact replication region.

7. Streptomyces ghanaensis DSM 2932.

**Claims for the contracting state AT:**

1. A process for the isolation of the plasmid pSG5 having a molecular length of about 12.7 kb, which is not cut by the restriction enzymes BglI, HpaI, HindIII and ClaI, is cut once by EcoRV, EcoRI, XhoI and BclI, and is cut twice by SphI and BglII, characterized by lysing a culture of Streptomyces ghanaensis DSM 2932 and obtaining the plasmid from the lysate.

2. The use of the plasmid pSG5 according to claim 1, or of its fragments containing the intact replication region, for the construction of plasmid vectors.

3. The use of the plasmid pSG5 according to claim 1, or of its fragments containing the intact replication region, for the construction of plasmid vectors for Streptomycetes.

4. The use of the plasmid pSG5 according to claim 1, or of its fragments containing the intact replication region, for the construction of shuttle vectors between Streptomycetes strains and other microorganisms.

**Revendications pour les Etats Contractants BE, CH, DE, FR, GB, IT, LI, LU, NL, SE**

1. Plasmide pSG5 ayant une longueur de molécule d'environ 12,7 kb, qui n'est pas coupé par les enzymes de restriction BglI, HpaI, HindIII et ClaI, est coupé une fois par EcoRV, EcoRI, XhoI et BclI, et deux fois par SphI et BglII, et peut être obtenu à partir d'une culture de Streptomyces ghanaensis DSM 2932.

2. Procédé pour l'isolement du plasmide pSG5 selon la revendication 1, caractérisé en ce qu'on lyse une culture de Streptomyces ghanaensis DSM 2932 et recueille le plasmide à partir du lysat.

3. Utilisation du plasmide pSG5 selon la revendication 1 ou de ses fragments contenant la région de réplication intacte, pour la construction de vecteurs plasmidiques.

4. Utilisation du plasmide pSG5 selon la revendication 1 ou de ses fragments contenant la région de réplication intacte, pour la construction de vecteurs plasmidiques pour des Streptomyces.

5. Utilisation du plasmide pSG5 selon la revendication 1 ou de ses fragments contenant la région de réplication intacte, pour la construction de vecteurs navette entre des souches de Streptomyces et d'autres micro-organismes.

6. Plasmides hybrides, qui contiennent le plasmide pSG5 selon la revendication 1 ou sa région de réplication intacte.

7. Streptomyces ghanaensis DSM 2932.

**Revendications pour l'Etat Contractant AT**

1. Procédé pour l'isolement du plasmide pSG5 ayant une longueur de molécule d'environ 12,7 kb, qui n'est pas coupé par les enzymes de restriction BglI, HpaI, HindIII et ClaI, est coupé une fois par EcoRV, EcoRI, XhoI et BclI, et deux fois par SphI et BglII, caractérisé en ce qu'on lyse une culture de Streptomyces ghanaensis DSM 2932 et recueille le plasmide à partir du lysat.

2. Utilisation du plasmide pSG5 selon la revendication 1 ou d'un fragment de ce plasmide contenant le réplicon intact, pour la construction de vecteurs plasmidiques.

3. Utilisation du plasmide pSG5 selon la revendication 1 ou d'un fragment de ce plasmide contenant le réplicon intact, pour la construction de vecteurs plasmidiques pour des Streptomyces.

4. Utilisation du plasmide pSG5 selon la revendication 1 ou d'un fragment de ce plasmide contenant le réplicon intact, pour la construction de vecteurs navette entre des souches de Streptomyces et d'autres micro-organismes.

FIG.1

pSG 5

FIG.2

pGM1
5,55kb

## FIG. 3

EcoRI  XhoI  PstI

SstII
ClaI
7
EcoRV
SalI  tsr
6
SstII

pGM 2
8,1 kb

Hind III
BglII
BclI
aphII
1
PstI

SphI

2

PstI
SalI
XhoI

3
SalI
SstII
SalI

5
SphI
SalI
PstI
4
SstII
PstI  BclI
PstI

## FIG. 4

EcoRI

5
0  Cmr
BclI 0,75

pACYC
184
1
EcoRV 1,15

pSLE41
5,4 kb
Thior

ClaI 1,45

4

Bcl I 1,8

3,75 ClaI
3,75 Hind III
3,6 EcoRV
3,4 BamHI
Tcr
2

3
3,2 SphI

# FIG. 5

pSW 344 E
18,1kb

pSLE 41

pACYC 184

pSG 5

# FIG. 6

pGM 101
9,85kb

FIG.7